Europäisches Patentamt

European Patent Office

Office européen des brevets

(19)

(11) Veröffentlichungsnummer: **0 053 794**
**A2**

(12) **EUROPÄISCHE PATENTANMELDUNG**

(21) Anmeldenummer: **81110069.2**

(22) Anmeldetag: **02.12.81**

(51) Int. Cl.³: **A 61 F 1/03**

(30) Priorität: **10.12.80 DE 3046553**

(43) Veröffentlichungstag der Anmeldung:
**16.06.82 Patentblatt 82/24**

(84) Benannte Vertragsstaaten:
**AT BE CH DE FR GB IT LI NL SE**

(71) Anmelder: **Feldmühle Aktiengesellschaft**
**Fritz-Vomfelde-Platz 4**
**D-4000 Düsseldorf 11(DE)**

(72) Erfinder: **Semlitsch, Manfred, Dr. Dipl.-Phys.**
**Endliker Strasse 86**
**CH-8404 Winterthur(CH)**

(72) Erfinder: **Dörre, Erhard, Dr. Dipl.-Phys.**
**Talweg 14**
**D-7310 Plochingen(DE)**

(72) Erfinder: **Neu, Kunibert, Dr. Dipl.-Ing.**
**Albstrasse 16**
**D-7312 Wangen(DE)**

(74) Vertreter: **Uhlmann, Hans, Dr. rer.nat., Dipl.-Chem.**
**Gladbacher Strasse 189**
**D-4060 Viersen 1(DE)**

(54) **Hüftgelenkpfanne.**

(57) Bei einer Endoprothese, bei der sowohl Gelenkkugel (5) als auch Hüftgelenkpfanne (1) aus oxidkeramischem Werkstoff bestehen, ist im Bereich der Öffnung der Hüftgelenkpfanne (1) eine kreisringförmige Ausnehmung (4, 4') angeordnet, in der sich ein Ring (6,6') aus bioinertem, plastisch verformbarem Material befindet. Die der Gelenkkugel zugewandte Oberfläche (13) des Ringes (6, 6') geht ohne wesentliche Unterbrechung völlig stufenlos in die kugelige Oberfläche (2) der Pfanne (1) über.

Im Fall einer Subluxation und einem damit verbundenen kurzzeitigen, linienförmigen Lagerkontakt (12) zwischen Gelenkkugel (5) und Gelenkpfanne (1) im Pfannenrandbereich resultiert selbst im Fall einer Trockenreibung eine günstige Tribologie zwischen der Gelenkkugel (5) und dem bei Subluxation damit in Eingriff stehenden Ring (6,6') aus plastisch verformbarem Material.

Fig. 1

EP 0 053 794 A2

- 1 -

Anmelder: Feldmühle Akteingesellschaft
Fritz-Vomfelde-Platz 4
D -4000 Düsseldorf 11

## Hüftgelenkpfanne

Die vorliegende Erfindung betrifft die Hüftgelenkpfanne einer Endoprothese, bei der sowohl die Gelenkkugel als auch die Gelenkpfanne aus oxidkeramischem
Werkstoff, insbesondere aus Aluminiumoxid bestehen.

Das Hüftgelenk ist maximalen Beanspruchungen unterworfen, die infolge der auftretenden Kräfte wesentlich höher als z.B. beim Schultergelenk sind. Der
Erfolg eines implantierten Hüftgelenkersatzes hängt
daher zu einem wesentlichen Teil vom Verschleiß der
miteinander in Eingriff stehenden Teile ab.

Man hat schon auf unterschiedliche Weise versucht,
den zwischen den einzelnen Gelenkteilen auftretenden
Verschleiß zu reduzieren und dadurch eine wesentliche
Voraussetzung für eine ausreichend lange Benutzungsdauer zu schaffen. Ein älterer, in der DE-AS 20 24 583
enthaltener Vorschlag sieht vor, zwischen Gelenkkugel
und -pfanne kleine Kugeln als Wälzkörper zu verwenden

- 2 -

und den Raum zwischen Gelenkkugel und -pfanne abzudichten, um ein Austreten von Schmiermitteln, wie z.B. Fett oder Teflonpulver zu vermeiden.

Nachteilig bei dieser vorzugsweise aus einer CR-CO-MO-Legierung hergestellten Hüftgelenkprothese ist neben der bei einer Metallprothese nicht mit Sicherheit auszuschließenden Metalloseerscheinung die komplizierte Konstruktion.

Auch Hüftgelenkpfannen aus Kunststoff, wie z.B. Polyäthylen, die üblicherweise mit einer Gelenkkugel aus Metall oder aus Aluminiumoxidkeramik kombiniert werden, haben trotz der relativ günstigen tribologischen Eigenschaften des Polyäthylens in vielen Fällen einen zu hohen Verschleiß. Solche Hüftgelenkpfannen werden in der DE-OS 26 28 530 und der DE-AS 25 27 865 vorgeschlagen.

Ein eigener Vorschlag der Anmelderin, wie er in der DE-OS 23 18 396 enthalten ist, sieht die Verwendung von miteinander in Eingriff stehenden Teilen einer aus hochreiner Aluminiumoxidkeramik hergestellten Hüftgelenkendoprothese vor. Vorteilhaft ist bei diesem Hüftgelenkersatz die erheblich verbesserte Körperverträglichkeit im Vergleich zu metallenen Gelenkteilen. Jedoch kann auch dieser Vorschlag noch nicht voll befriedigen. Die hervorragenden Verschleißeigenschaften zweier miteinander in Eingriff stehender und auf Reibung beanspruchter Teile aus Aluminiumoxidkeramik bleiben nämlich nur so lange erhalten, wie zwischen diesen beiden Teilen ein ausreichender Schmierfilm vorliegt. Kommt es dagegen zu einer

- 3 -

Zerstörung des Schmierfilms, nimmt der Verschleiß infolge der dann einsetzenden Trockenreibung rapide zu. Zu Zerstörungen des Schmierfilms kommt es, wenn die auftretenden Belastungen bestimmte Werte überschreiten, was beispielsweise durch Reduzierung der Kontaktfläche erfolgen kann und wobei die spezifische Flächenbelastung erhöht wird. Dabei wird der Schmierfilm weggedrückt und es kommt zur Trockenreibung einschließlich der oben aufgeführten Nachteile, insbesondere zu einem erhöhten Verschleiß. Bei einer Hüftgelenkprothese tritt dieser Fall dann auf, wenn die Kugel bei Bewegung nicht mehr zentral in die Pfanne eingreift, sondern nur noch mit dem Pfannenrand in Berührung kommt, so daß es statt zu einer flächigen zu einer linienförmigen Berührung kommt. Dieses teilweise Auskugeln der Gelenkkugel wird als Subluxation bezeichnet. Ursachen für eine Subluxation können beispielsweise eine zu geringe Muskelspannung, eine nicht optimale Positionierung der künstlichen Gelenkpfanne oder ein Heraushebeln der Kugel sein. Der letztere Fall tritt vor allem dann ein, wenn aus anatomischen Gründen eine Kugel mit Halsteil verwendet wird.

Die infolge einer Subluxation auf einer reduzierten Kontaktfläche auftretenden Belastungskräfte sind in der Regel so stark, daß sie ein Wegdrücken des aus körpereigener Flüssigkeit gebildeten Schmierfilms bewirken und damit zur Trockenreibung führen.

- 4 -

Die optimale Tribologie eines Hüftgelenkersatzes, bei dem Gelenkpfanne und Gelenkkopf aus hochreiner Aluminiumoxidkeramik bestehen, wird dadurch in erheblicher Weise verschlechtert und an den Stellen, bei denen eine Linienberührung stattfindet, kann es zu signifikanten Verschleißerscheinungen kommen.

Gemäß der DE-OS 29 25 089 wurde auch bereits bei einem künstlichen Hüftgelenk vorgeschlagen, an der Öffnung einer Gelenkpfanne einen Rückhaltering anzuordnen, um dadurch das Auskugeln der Gelenkkugel zu verhindern. Der Rückhaltering ist dabei so ausgebildet, daß er die Gelenkpfannenaushöhlung verlängert und zwar über den Äquator der Gelenkkugel bzw. der Gelenkpfannenaushöhlung. Nachteilig bei diesem System ist es, daß eine Subluxation durch die formschlüssige Fixierung der Gelenkkugel in der Gelenkpfanne zwar verhindert wird, durch diese Konstruktion aber andererseits die bei einer Subluxation auftretenden Kräfte lockernd auf die auf einem Schaft befestigte Gelenkkugel einwirken können.

Ein Vorschlag zur dringend anstehenden Lösung der tribologischen Probleme im Pfannenrandbereich bei aus Aluminiumoxid hergestellten Hüftgelenk-Endoprothesen kann der DE-OS 29 25 089 noch nicht entnommen werden.

Die Aufgabe der vorliegenden Erfindung liegt in der Beseitigung der bestehenden Nachteile. Insbesondere sollen bei einer Hüftgelenkprothese, bei der sowohl die Gelenkkugel als auch die Hüftgelenkpfanne selbst aus oxidkeramischen Werkstoffen, wie z.B. aus hochreiner Aluminiumoxidkeramik bestehen, die bei einer Subluxation auftretenden Nachteile beseitigt werden. Vorrangig soll der erhöhte Verschleiß im Bereich der Pfannenöffnung, der infolge einer durch Subluxationen hervorgerufenen Linienberührung, bzw.der dabei erfolgenden Verdrängung des Schmiermittelfilms durch die Trockenreibung der Gelenkteile entsteht, eingeschränkt werden.

Zur Lösung dieser Aufgabe sieht die Erfindung eine Hüftgelenkpfanne einer Hüftgelenkprothese vor, bei der sowohl die Gelenkkugel als auch die Hüftpfanne selbst aus oxidkeramischem Werkstoff, insbesondere aus Aluminiumoxid, bestehen und die dadurch gekennzeichnet ist, daß die Hüftgelenkpfanne im Bereich ihrer Öffnung eine kreisringförmige Ausnehmung aufweist, in der sich ein Ring aus bioinertem, plastisch verformbarem Material befindet, dessen der Gelenkkugel zugewandte Oberfläche ohne wesentliche Unterbrechung völlig stufenlos in die kugelige Oberfläche der Pfanne übergeht.

Durch die erfindungsgemäße Ausbildung der Hüftgelenkpfanne wird bei einer eventuell auftretenden Subluxation vermieden, daß es im Bereich der Pfannenöffnung zu einem Kontakt zwischen dem oxidkeramischen Werkstoff der Hüftgelenkpfanne und dem gleichen Werkstoff der Gelenkkugel kommt. Die Anordnung eines Ringes aus plastisch verformbarem Material stellt dabei sicher, daß selbst dann, wenn es im Pfannenrandbereich zur Trockenreibung kommt, der Verschleiß in sehr geringen Grenzen gehalten werden kann.

- 6 -

Der durch die Wahl von Polyäthylen als Material zur Herstellung des Ringes erzielbare Vorteil erklärt sich dadurch, daß bei Trockenreibung zwischen Keramik und Polyäthylen wesentlich geringerer Verschleiß auftritt als zwischen zwei Werkstoffteilen aus Keramik, die auf Trockenreibung beansprucht werden. Während vollständig aus Polyäthylen hergestellte Hüftgelenkpfannen vielfach über eine zu geringe Verschleißfestigkeit verfügen, wirkt sich die Wahl von Polyäthylen als Ringmaterial im vorliegenden Fall deswegen nicht nachteilig aus, weil der Ring aus Polyäthylen immer nur sehr kurzzeitig belastet wird, nämlich nur dann, wenn tatsächlich eine Subluxation auftritt.

Weitere vorteilhafte Ausführungsformen der vorliegenden Erfindung sind durch die Merkmale der Unteransprüche gekennzeichnet.

Weitere Einzelheiten und Vorteile der Erfindung ergeben sich aus der Beschreibung von beispielsweisen Ausführungsformen der Erfindung an Hand der Figuren 1 bis 8.

Es zeigen:

Fig. 1 ein erstes Ausführungsbeispiel einer Hüftgelenkpfanne mit Gelenkkugel,

Fig. 2 eine weitere Ausführungsform, bei welcher ein mehrteiliger Ring zur Anwendung kommt,

Fig. 3 eine vergrößerte Detaildarstellung zum Bereich III der Fig. 2,

Fig. 4 und 5   das äußere Ringteil nach Fig. 2,
zweigeteilt in Schnitt, gemäß der Linie
IV - IV in Fig. 5 , und Aufsicht,

Fig. 6 eine Darstellung der Fig. 4 entsprechend
zeigend das innere Ringteil,

Fig. 7 und 8   das innere Ringteil als zweiteilige
Ausführung in Schnitt, gemäß der Linie
VII - VII in Fig. 8,  und Aufsicht.

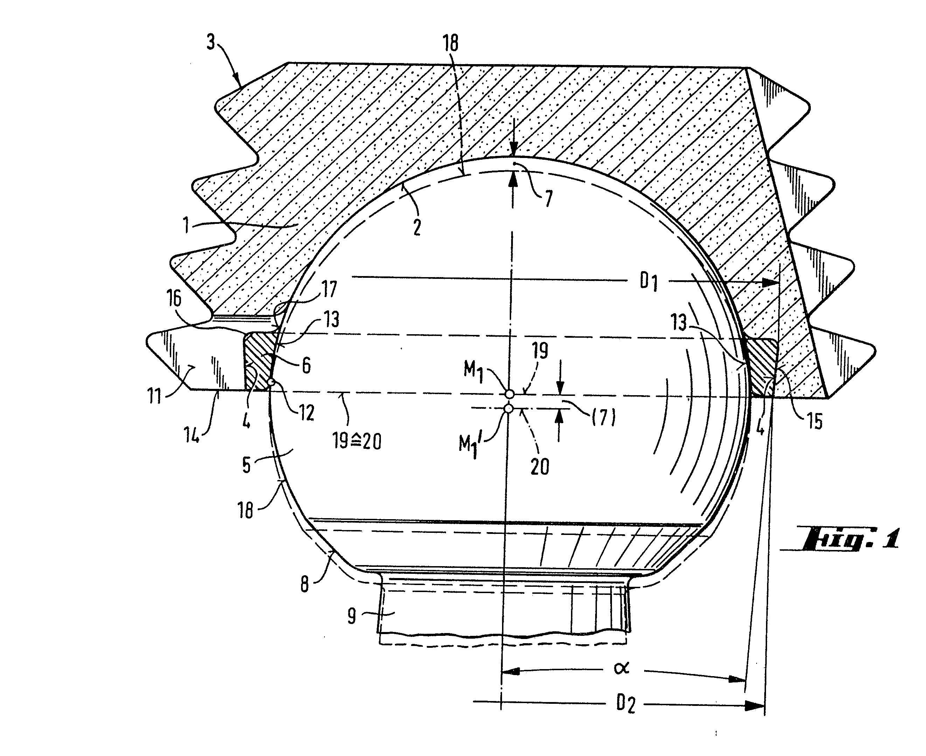

Die Hüftgelenkpfanne 1 der Fig. 1, bestehend aus
Aluminiumoxid, weist eine konkave Ausnehmung auf,
welche eine kugelige Pfannenoberfläche 2 zur gleitlagernden Aufnahme einer Gelenkkugel 5 hat. Die
gezeigte Ausführungsform weist zur Befestigung der
Hüftgelenkpfanne 1 im Beckenknochen ein in an sich
bekannter Weise ausgebildetes Spitz-Rund-Gewinde 3
auf, das vorzugsweise selbstschneidend durch Eingriff
eines Werkzeuges in die Werkzeugnuten 11 eingeschraubt wird.

An dem mit 8 bezifferten konischen Bereich am Ende
der Gelenkkugel 5 ist das mit 9 bezeichnete Ende
eines Schaftes, auf dem die Gelenkkugel 5 befestigt
ist, erkennbar.

Die Hüftgelenkpfanne 1 weist ferner an ihrem äußeren
Rand eine kreisringförmige Ausnehmung 4 auf zur Aufnahme eines Ringes 6 aus bioinertem Material, nämlich
aus hochmolekularem Niederdruckpolyäthylen. Um diesen
gegen Herausfallen zu sichern, ist der äußere Mantel
15 des Ringes 6 bzw. die kreisringförmige Ausnehmung
4 konisch ausgebildet  (Winkel $\alpha > 0°$, vorzugsweise
3 bis 8°), so daß der innere Durchmesser $D_1$ größer

als der äußere Durchmesser $D_2$ ausfällt und damit ein formschlüssiger Halt des Ringes 6 in der Hüftgelenkpfanne 1 gegeben ist.

Die kreisringförmige Ausnehmung 4 und der Ring 6 sind so dimensioniert, daß die Innenoberfläche 13 des Ringes 6 völlig stufenlos in die kugelige Oberfläche 2 der Pfanne 1 übergeht.

An der kreisringförmigen Ausnehmung 4 sind Radien 16, 17 vorgesehen, um die Gefahr von Ausbrüchen zu vermeiden, wie sie bei scharfen Kanten gegeben ist.

Bei Subluxation entsteht eine Distanz 7 zwischen der Kugeloberfläche 18 und der Pfannenoberfläche 2. Dabei gelangt die Kugel 5 von einem flächigen Lagerkontakt in einen linienförmigen Lagerkontakt 12 im Bereich ihres Äquators 20 mit dem Ring 6 aus bioinertem Material.

Bei dieser Ausführung ist die Außenkontur 14 der Hüftgelenkpfanne 1 bzw. des Ringes 6 in etwa zusammenfallend mit dem Äquator 20 der Gelenkkugel 5 und dem Äquator 19 der kugeligen Pfannenoberfläche 2.

Das Zusammenhalten der Kugel 5 mit der Hüftgelenkpfanne 1 erfolgt hier also kraftschlußbedingt durch Muskelspannung.

$M_1$ stellt den Mittelpunkt der Gelenkkugel 5 und der kugeligen Pfannenoberfläche 2 in Normallagerzustand dar, wobei beide Mittelpunkte hier in $M_1$ zusammenfallen. $M_1'$ ist die Lage des Mittelpunktes der Gelenkkugel 5 bei Subluxation.

- 9 -

Der Vorteil der in Fig. 1 gezeigten, ganz besonders bevorzugten Ausführungsform ist wie folgt begründet: Durch die maximal halbkugelförmige Ausbildung der Pfannenoberfläche 2 sind die kreisringförmige Ausnehmung 4 bzw. der Ring 6 oberhalb der Äquatore der Gelenkkugel 5 bzw. der Pfannenoberfläche 2 angeordnet, d.h. sie übergreifen die Äquatore in Richtung des Gelenkkugelschaftes nicht. Tritt nun tatsächlich eine Subluxation auf, besteht im Gegensatz zu einer Ausführungsform, bei der die Gelenkkugel formschlüssig in der Gelenkpfanne gehalten wird, nicht die Gefahr, daß die auftretenden Zugkräfte lockernd auf die Gelenkkugel einwirken und diese unter Abrieberzeugung vom üblicherweise aus Metall hergestellten Schaft abziehen.

Es liegt auch im Bereich der Erfindung, daß die kreisringförmige Ausnehmung 4 bzw. der Ring 6 die Äquatore 19, 20 in Richtung des Schaftes geringfügig überschreiten, jedoch ist die oben beschriebene - die Äquatore nicht überschreitende Ausführungsform - die besonders bevorzugte.

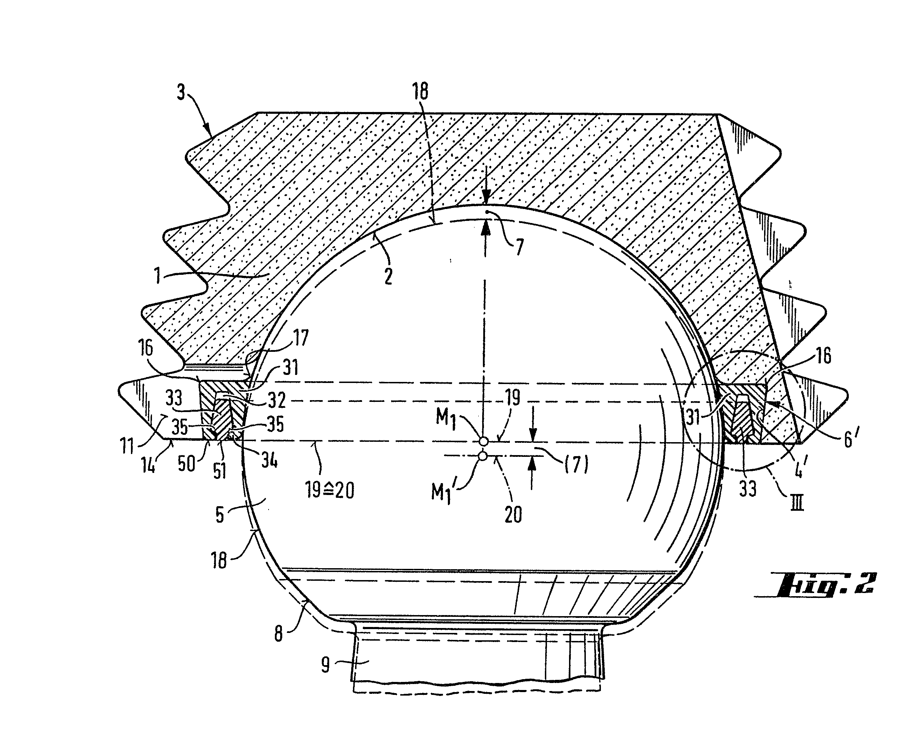

Die Fig. 2 zeigt die Hüftgelenkpfanne 1 nach Fig. 1 bestückt mit der Gelenkkugel 5 und einem mehrteiligen Ring 6', der in eine entsprechende Ausnehmung 4' eingesetzt ist. Dieser ist wieder aus bioinertem plastisch verformbarem Material hergestellt und besteht aus einem im Querschnitt V-förmigen Lagerring 31, welcher umlaufend und einstückig ausgebildet ist, und einem Indexring 33, der in eine im Querschnitt keilförmig ausgebildete umlaufende Öffnung in Form einer Nut 32 einsetzbar ist. Wie spätere Ausführungen noch zeigen, kann der Lagerring 31 auch ein- oder mehrfach geteilt ausgebildet sein.

Der Indexring 33 ist gegen Herausfallen durch Krägen 34 am Ring 31 einerseits und durch Schultern 35 am Indexring 33 andererseits gesichert. Vorzugsweise ist der Indexring 33 so ausgeführt, daß seine untere Kante 51 bündig mit der unteren Kante 50 des Lagerringes 31 abschließt.

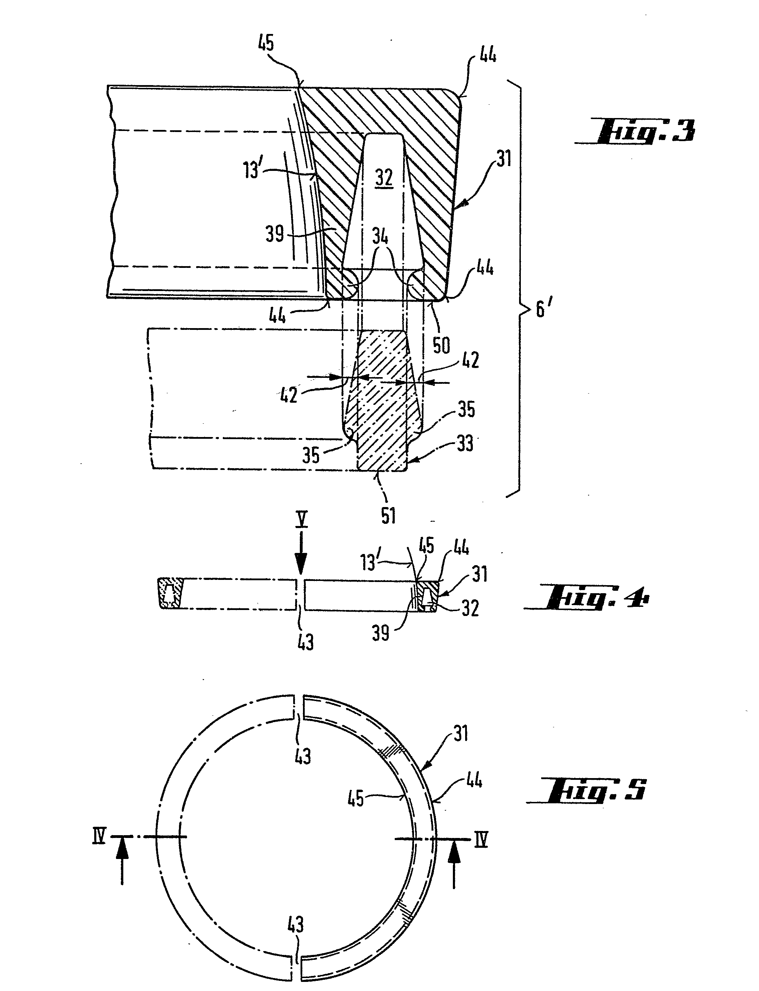

Fig. 3 zeigt, daß die der Gelenkkugel 5 zugewandte Seite 13' dieses Lagerringteiles 31 am inneren Ringbereich 39 von gleicher Kugelgestalt ist, wie die Pfannenoberfläche 2 und in diese harmonisch und stufenlos überläuft.

Der Ring 31 ist in seinem Querschnitt überall gerundet ausgeführt, so daß er Rundungen 44 aufweist, jedoch mit Ausnahme der Kante 45. Hier ist er so scharfkantig wie möglich gehalten, um zwischen der Pfannenoberfläche 2 und dem Ring 6' keinen Spalt entstehen zu lassen.

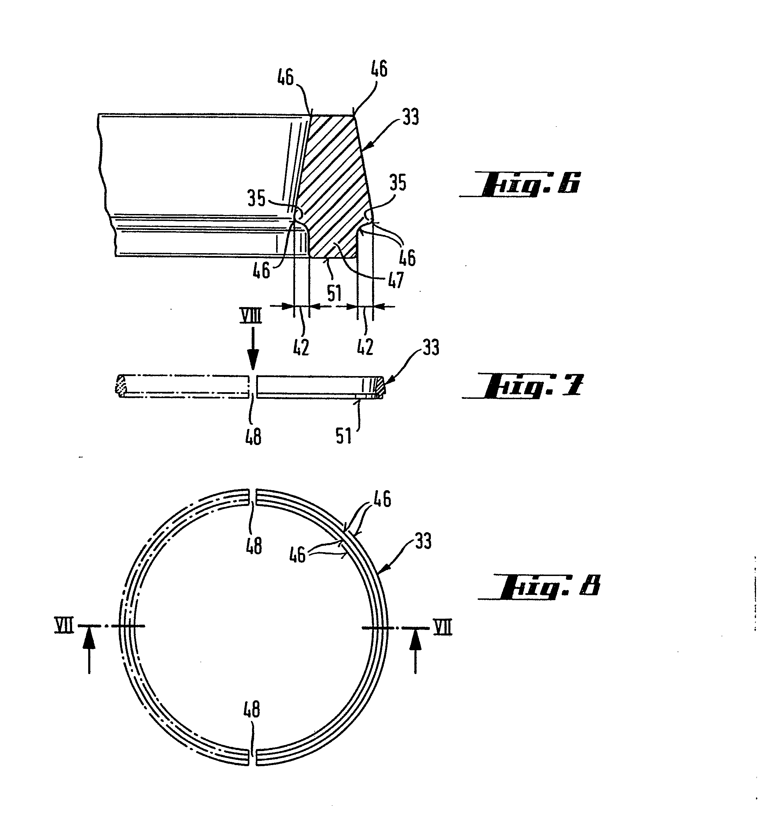

Wie Fig. 4 und 5 zeigen, kann der Lagerring 31 durch eine Trennfuge 43 mittig geteilt ausgeführt sein. Es liegt auch im Bereich der Erfindung, diesen Ring 31 auch drei- oder vierfach zu teilen.

Ähnliches gilt für den Indexring nach Fig. 6, welcher Rundungen 46 aufweist. Der Halsteil 47 wird rundumlaufend von den Schultern 35 mit dem Überdeckungsmaß 42 überragt.

Auch der Indexring 33 kann durch Trennfugen 48 zwei- oder mehrfach geteilt ausgeführt sein, wie die Fig. 7 und 8 veranschaulichen.

-12-

Patentansprüche

1. Hüftgelenkpfanne einer Endoprothese, bei der sowohl die Gelenkkugel als auch die Gelenkpfanne aus oxidkeramischem Werkstoff, insbesondere aus Aluminiumoxid bestehen, gekennzeichnet dadurch, daß die Hüftgelenkpfanne (1) im Bereich ihrer Öffnung eine kreisringförmige Ausnehmung (4,4') aufweist, in der sich ein Ring (6,6') aus bioinertem, plastisch verformbarem Material befindet, dessen der Gelenkkugel zugewandte Oberfläche (13) ohne wesentliche Unterbrechung völlig stufenlos in die kugelige Oberfläche (12) der Pfanne (1) übergeht.

2. Hüftgelenkpfanne nach Anspruch 1, dadurch gekennzeichnet, daß der Ring (6) dadurch im Klemmsitz gehalten wird, daß der an der Pfannenöffnung liegende Durchmesser $D_2$ des Ringes (6) und der kreisförmigen Ausnehmung (4) kleiner ist als der innenliegende Durchmesser $D_1$.

3. Hüftgelenkpfanne nach einem der Ansprüche 1 und 2, dadurch gekennzeichnet, daß der Ring (6') aus einem Lagerring (31) und einem Indexring (33) besteht.

4. Hüftgelenkpfanne nach einem der Ansprüche 1 bis 3, dadurch gekennzeichnet, daß der Lagerring (31) einen V-förmigen Querschnitt hat

5. Hüftgelenkpfanne nach einem der Ansprüche 1 bis 4, dadurch gekennzeichnet, daß der Lagerring (31) eine im Querschnitt keilförmig ausgebildete umlaufende Öffnung in Form einer Nut (32) zur Aufnahme des Indexringes (33) aufweist.

6. Hüftgelenkpfanne nach einem der Ansprüche 1 bis 5, dadurch gekennzeichnet, daß der Indexring (33) zwei oder mehrere Schultern (35) zur Auflage auf Krägen (34) des Lagerringes (31) aufweist.

7. Hüftgelenkpfanne nach einem der Ansprüche 1 bis 6, dadurch gekennzeichnet, daß der Lagerring (31) durch eine Trennfuge (43) mittig geteilt ist.

8. Hüftgelenkpfanne nach einem der Ansprüche 1 bis 7, dadurch gekennzeichnet, daß der Indexring (33) durch eine Trennfuge (48) mittig geteilt ist.

**Fig. 1**

Fig. 2

*Fig. 3*

*Fig. 4*

*Fig. 5*

**Fig. 6**

**Fig. 7**

**Fig. 8**